# EUROPEAN PATENT SPECIFICATION

(11) **EP 0 388 356 B1**
(45) Date of publication and mention of the grant of the patent: **08.07.1998**
(21) Application number: 90810174.4
(22) Date of filing: 07.03.1990
(51) Int. Cl.: C07D 251/44, C07D 251/50, C07D 403/12, G02C 7/10

(54) **Ultraviolet absorbing lenses and methods of making the same**
Ultraviolett absorbierende Linse und Verfahren zu deren Herstellung
Lentilles absorbant l'ultraviolet et leurs méthodes de fabrication

(30) Priority: 14.03.1989 US 323327; 14.03.1989 US 323354
(43) Date of publication of application: 19.09.1990
(73) Proprietor: Novartis AG, 4058 Basel (CH)
(72) Inventor: Hung, William M., Alpharetta, Georgia 30201 (US); Su, Kai C, Alpharetta, Georgia 30201 (US)

(56) References cited:
- US-A- 3 055 896
- US-A- 4 390 676
- US-A- 4 528 311
- US-A- 4 559 059

## Description

The present invention relates to ultraviolet radiation absorbing agents, to ocular lenses, e.g. contact lenses or intraocular lenses to which said agents are bonded and to a method for the preparation of such lenses. More particularly, the invention relates to hydrophilic or soft contact lenses having a reactive ultraviolet radiation absorbing agent covalently bonded to polymeric material.

Ultraviolet radiation is ever present in our environment, and consists of wave lengths between 200 and 400 nm. Exposure to ultraviolet radiation has been found to be the cause of several ocular pathologies. The damaging effect of ultraviolet radiation on the corneal epithelium has been known for a long time. For instance, studies have demonstrated the damaging effect of 290 nm radiation on the rabbit corneal epithelium (A.P. Cullen, Ultraviolet Induced Lysosome Activity in Corneal Epithelium, Graefes Arch. Clin. Exp. Ophthalmol 214; 107-118, 1980), as well as changes in the stroma and endothelium of primary corneal layers (epithelium, stroma and endothelium) subsequent to exposure to a commercially available UV suntan lamp which emits radiation across the full spectrum from 280 nm (A. Ringvold et al., Changes in the Rabbit Corneal Stroma Caused by UV-Radiation, Acta Ophthalmol. (Copenhagen) 63; 601-606, 1985). Compounding the damage is the fact that ultraviolet radiation damage to the eye is known to be cumulative and obeys the law of reciprocity. These findings reinforce the importance of adequate ocular protection against ultraviolet radiation. Such protection is particularly ended for people who are prone to UV exposure, patients who have had cataract and patients on photo-sensitizing drugs.

Recently, contact lenses have been developed which serve to absorb ultraviolet radiation. For example, U.S. Patent No. 4,390,676 discloses an ultraviolet absorbing contact lens formed by copolymerizing a monomer suitable for making lenses and an ultraviolet absorber for absorbing radiation having wavelengths of 340 to 450 nm. The UV absorbing compound, 2-hydroxy-4-methacryloxy-benzophenone or mixtures thereof, is incorporated into the lens polymeric material at the molecular level. Also, U.S. Patent No. 4,528,311 discloses ultraviolet light absorbing contact lenses made of a polymeric composition comprising copolymers of 2-hydroxy-5-acrylyloxyphenyl-2H-benzotriazole with one or more other monomers copolymerizable therewith.

The above compounds have been found to copolymerize and give protection to the material. However, the copolymerization efficiency of the compounds has proved to be inadequate. Typically, no more than 15 % of the alkenyloxy-benzophenones actually become part of the polymeric chain. The remainder of the material is easily leached out by solvent extraction. Furthermore, while the hydroxy benzophenones copolymerizable with acrylate monomers are effective UV absorbers and form chemically stable copolymers, relatively large amounts, i.e. 3 to 10 % by weight, must be incorporated in the polymer to obtain 85 % UV absorption at 400 nm and 1 mm thickness. Also, the compounds exhibit very broad absorption bands which extend into the visible spectrum, and lenses incorporating these ingredients tend to be unacceptably yellow in color.

The above described UV absorbing lenses also possess several limitations. For instance, the absorbing agents and the lens material have different properties, and only one absorbing agent is used and appears symmetrically as a thick film on the lens. As a result, the lenses have structural weaknesses and exhibit inconsistent expansion, which in turn results in overly curved or otherwise misshapened lenses. Furthermore, the application of the agent to the lens takes a relatively long time, must be done at high temperature, and requires a high concentration of the expensive agent. Also, the use of a single absorbing agent limits the range of UV wavelengths which the lens may absorb.

There exists, therefore, a need for improved ultraviolet radiation absorbing contact lenses, as well as a method for their production.

There also exists a need for such lenses which have structural integrity, which can be prepared in relatively short time and at relatively low temperatures and which use small amounts of UV absorbing agents.

There exists a further need for such lenses which absorb a broad range of UV wavelengths.

There also exists a more particular need for a lens which incorporates a relatively small amount of absorbing agent, which exhibits relatively little yellowing, and from which the absorbing agent does not leach out.

The present invention relates to ultraviolet radiation absorbing lenses of a polymeric lens material characterized in that a UV radiation absorbing agent is covalently bonded to the polymeric lens material, and to a method for the manufacture thereof. The lens exhibits very little yellowing, and can be produced using a relatively small amount of the absorbing agent. Also, because of the covalent bonding, the absorbing agent does not leach from the lens.

The ultraviolet radiation absorbing agent has the formula I 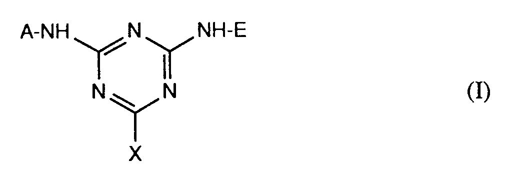 wherein A is an ultraviolet radiation absorbing group; E is an aqueous soluble group E₁ and X is Cl or F according to formula (IB), or E is an ultraviolet radiation absorbing group E₂ and X is Cl according to formula (IA) ; 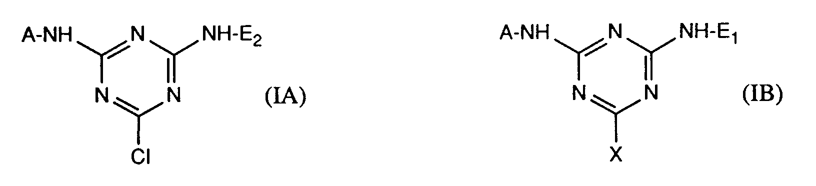 wherein A and E₂ are either identical to or dissimilar to each other and wherein A and E₂ are selected from the radicals of benzoic acid, salicylic acid, substituted benzophenone and substituted benzotriazoles, benzoic acid esters, acrylates, oxalic acid diamides, and hydroxy phenyltriazines; and
wherein E₁ is an aqueous soluble group represented by the radical of the formula 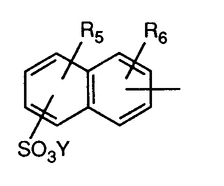 wherein Y is hydrogen, R₅ and R₆ are hydrogen, C₁-C₁₈alkyl, C₁-C₁₈alkoxy, halogen, nitro, hydroxy, carboxy or sulfo and salts of said radicals;
with the proviso that A and E₂ are not simultaneously selected from 2-(o-hydroxyaryl)benzotriazole.

The agent should be water soluble because the step of incorporating the agent onto the lens material is performed in an aqueous medium.

The UV radiation absorbing groups A and E₂ may be identical or different. If A and E₂ absorb radiation of different wavelengths, a lens having the agent with both groups will be capable of absorbing radiation having wavelengths of the union of A and E₂. Also, regardless of whether the A and E₂ groups are similar or different, less agent will be needed on the lens when both A and E₂ are ultraviolet absorbing groups.

It has been found that adding two different UV absorbing components onto the agent can provide a contact lens which will absorb a wide range of wavelengths. For example, the absorbing component Tinuvin® P (available from Ciba-Geigy Corporation), which is a benzotriazole type absorber, blocks UV radiation from about 280 nm to 360 nm very well, but it does not block well at about 250 nm to 275 nm. The 4-aminobenzoic acid type component blocks radiation from 190 nm to 316 nm very well, but little higher. By combining the two components onto a single molecule according to the present invention, the resultant UV absorbing agent, and hence a lens incorporating the agent, will have excellent UV absorption from about 190 nm to 360 nm (the union of the spectra of the two components).

Similarly, when a benzophenone type component (which also blocks UV radiation from about 280 nm to 360 nm) is added to 4-aminobenzoic acid type component in place of the Tinuvin® P above, the resulting reactive UV absorbing agent will have excellent UV absorption from about 190 nm to 360 nm.

It is also possible to add similar or identical UV absorbing components onto the agent. In such a case, the amount of agent needed to provide an effective radiation absorbing lens will be decreased. The decrease of agent on the lens greatly reduces the structural weaknesses associated with lenses having single-component absorbing agents.

It has also been found that the absorbing agent of the present invention can be applied to a lens at about room temperature and in a relatively short time by simply dipping or otherwise placing the lens into an aqueous medium having the agent dissolved therein.

This enables the agent to be applied to the lens by an optometrist at the point of purchase, rather than at the facility where the lens is made. Therefore, the optometrist does not need to maintain a large inventory of already absorbent lenses.

While the present invention is applicable to intraocular lenses and lenses used in spectacles, it will be described in connection with contact lenses. The present invention relates to lenses having a UV radiation absorbing agent bonded to its polymeric lens material. The absorbing agent is water soluble and has a molecular structure which contains one or two UV radiation absorbing groups thereon which may either be the same or different from each other. The agent is bound to the polymeric lens material exoskelatally.

The ultraviolet radiation absorbing groups A and E₂ may be the same or different and are selected from suitable radicals of essentially any UV absorbing compounds; however, if both A and E are UV absorbing groups, at least one of them should bring the structure of formula I water solubility.

Typical radicals of UV absorbers are those radicals of UV absorbers disclosed in the following patents, all of which are incorporated herein by reference: U.S. 3,041,330; 3,159,646; 3,213,058; 3,214,436; 4,418,000; 4,418,001; 4,418,002; 4,826,978; 3,493,539; 3,399,173; 4,880,859; 4,785,063; DEOS 1,495,870; GB 981,539 and EP-A-133,164.

The ultraviolet radiation absorbing groups A and E₂ are preferably selected from radicals having any of the following structures: 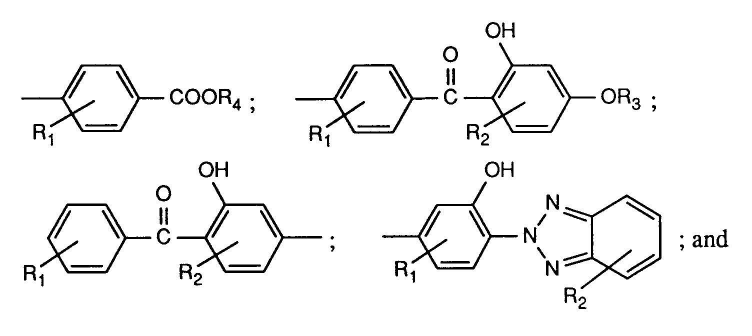 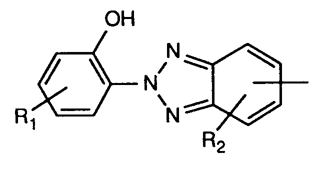 wherein R₁ and R₂ are independently of each other hydrogen, C₁-C₁₈alkyl, C₁-C₁₈alkoxy, halogen, nitro, hydroxy, carboxy or sulfo; and R₃ and R₄ are hydrogen or C₁-C₁₈alkyl; and salts thereof.

C₁-C₁₈Alkyl preferably has up to 7 carbon atoms, more preferred up to 4 carbon atoms and is e.g. methyl, ethyl, propyl, n-butyl, t-butyl, octyl or dodecyl.

C₁-C₁₈Alkoxy preferably has up to 7 carbon atoms, more preferred up to 4 carbon atoms and is e.g. methoxy, ethoxy, propoxy, t-butyloxy, octyloxy or dodecyloxy.

Halogen is e.g. chloro, fluoro or bromo.

Salts of the groups A and E₂ are preferably salts of radicals where R₁ and/or R₂ are sulfo, such that sulfonic acid salts result, e.g. alkali metal salts, such as sodium sulfonates.

Preferred are said radicals wherein said alkyl or alkoxy have 1 to 4 carbon atoms.

Another group of UV radiation absorbers of interest includes benzoic acid esters, cyano and carbomethoxy acrylates, oxalic acid diamides, and hydroxyphenyltriazines.

Particularly suitable for use in the instant invention as UV radiation absorber groups are the radicals of the benzophenones and benzotriazoles. Also of particular interest are the radicals of p-benzoic and salicylic acids such that e.g. A-NH- of formula I is the radical of p-amino(benzoic or salicylic) acid). More specifically, the UV radiation absorbers of interest include:
oxalic acid diamides, such as diamides of oxalic acid with anilines which are substituted by one or more substituents selected from C₁-C₁₂alkoxy or C₁-C₈alkyl, or with di-(C₁-C₄alkyl)amino-C₁-C₆alkyl amines, for example 4,4'-dioctyloxyoxanilide, 2,2'-dioctyloxy-5,5'-di-tert-butyloxanilide, 2,2'-didodecyloxy-5,5'-di-tert-butyloxanilide, 2-ethoxy-2'-ethyloxanilide, N,N'-bis(3-dimethylaminopropyl)oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy-disubstituted oxanilides and mixtures of o- and p-ethoxy-disubstituted oxanilides;
2-(2-hydroxyphenyl)-1,3,5-triazines, substituted by one or more substituents selected from C,-C₁₀alkyl, C₁-C₁₂alkoxy or C₁-C₄alkyl substituted phenyl, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine;
2-(2'-hydroxyphenyl)benzotriazoles, substituted by one or more substituents selected from C₁-C₇alkyl, C₁-C₁₀alkoxy, halogen or phenyl-C₁-C₄alkyl, for example the 5'-methyl, 3',5'-di-tert-butyl, 5'-tert-butyl, 5'-(1,1,3,3-tetramethylbutyl), 5-chloro-3',5'-di-tert-butyl, 5-chloro-3'-tert-butyl-5'-methyl, 3'-sec-butyl-5'-tert-butyl, 4'-octoxy, 3',5'-di-tert-amyl and 3',5'-bis(α,α-dimethylbenzyl) derivatives;
2-hydroxybenzophenones, substituted by one or more substituents selected from hydroxy, C₁-C₁₂alkoxy or phenyl-C₁-C₄alkoxy, for example the 4-hydroxy, 4-methoxy, 4-octoxy, 4-decyloxy, 4-dodecyloxy, 4-benzyloxy, 4,2',4'-trihydroxy and 2'-hydroxy-4,4'-dimethoxy derivatives;
esters of hydroxy and/or C₁-C₄alkyl substituted and unsubstituted benzoic acids, with phenols or C₁-C₈alkyl substituted phenols or C₁-C₁₈alkanols, for example, 4-tert-butylphenyl salicylate, phenyl salicylate, octylphenyl salicylate, dibenzoylresorcinol, bis(4-tert-butylbenzoyl)-resorcinol, benzoylresorcinol, 2,4-di-tert-butylphenyl 3,5-di-tert-butyl-4-hydroxybenzoate and hexadecyl 3,5-di-tert-butyl-4-hydroxybenzoate and acrylates with C₁-C₁₀alkanols, substituted by cyano or C₁-C₄alkoxycarbonyl in the α-position and substituted in the β-position by at least one phenyl, C₁-C₄alkoxyphenyl or indoline, the other β-position being unsubstituted or substituted by C₁-C₄alkyl, phenyl or C₁-C₄alkoxyphenyl, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxycinnamate, methyl α-carbomethoxy-p-methoxycinnamate and N-(β-carbomethoxy-β-cyanovinyl)-2-methylindotine.

Particularly preferred are the compounds of the examples.

Salts of groups E₁ are e.g. salts of radicals where R₅ and/or R₆ are sulfo, such that sulfonic acid salts result, e.g. alkali metal salts, such as the sodium salts, or salts with ammonia, e.g. ammonium salts, or with an amine, e.g. a lower alkyl amine, a di- or tri-lower alkyl-amine, or a phenyl-lower alkyl amine. Lower alkyl defines groups having up to seven, preferably up to 4 carbon atoms, e.g. methyl, ethyl or propyl. Suitable amines for salt formation are e.g. triethyl amine or benzyl amine. Even Y may be, instead of hydrogen, such salt forming group, e.g. an alkali metal such as sodium or amino group as hereinbefore defined.

Other solubilizing groups will be apparent to those of ordinary skill from the dyestuff art.

The compounds of formula I can be prepared by reacting compounds A-NH₂ and E-NH₂ with cyanuric acid trihalide. Basically, mono amine substituted UV radiation absorber is reacted with a trihalo triazine. The product is then reacted with either another amino-substituted UV absorber or a solubilizing moiety. Alternatively, an amino-substituted solubilizing moiety can be reacted with the trihalo triazine first and then an amino-substituted UV absorber is reacted with the product. The process will be more specifically seen with reference to the benzophenones in the examples.

The ultraviolet radiation absorbing components or aqueous soluble components represented by NH₂A or NH₂E respectively which are required to obtain the mono-halo-s-triazine of formula I belong to known classes of compounds and are readily obtained by conventional procedure well known in the art, e.g. such as the ones described in U.S. Patent Nos. 3,159,646 and 3,041,330.

The compounds of the polymeric lens material may vary so long as there is present in the monomer mixture a component which will provide the polymer with the required exoskeletal functional groups. The required exoskeletal functional groups include any group that is coreactive with a halogen substituted triazine such that a covalent bond is formed between the lens material and the triazine molecule, usually with the elimination of hydrogen halide. Such groups, prior to reacting include to hydroxy, amino, amido, mercapto, carboxy.

Monomers having the above groups which are suitable for use in the invention include: hydroxyalkyl esters of polymerizable unsaturated acids, such as acrylic, methacrylic, fumaric, maleic; unsaturated acids per se, such as acrylic, methacrylic, fumaric, maleic; heterocyclic N-vinyl lactams, such as N-vinyl pyrrolidone; noncyclic amides such as N-(1,1-dimethyl-3-oxobutyl)-acrylamide; amino alkyl esters of unsaturated acids such as 2-aminoethylacrylate, methacrylate, fumarate, or maleate; mercapto alkyl esters of unsaturated acids such as 2-mercapto ethyl acrylate, methacrylate, fumarate or maleate.

Other suitable monomers and reactive groups suitable for reacting with the halotriazine will be apparent for those of ordinary skill.

In addition to the monomers having the required halotriazine coreactive groups, the lens material may have a number of other monomeric components which do not have the stated reactive groups or such groups serve other purposes as when such a monomer is utilized as a crosslinking agent. The monomer, once crosslinking has taken place, is generally not available for interaction with the halotriazine, However, if more than two suitable reactive groups are present, such a monomer may indeed provide suitable reactive sites for covalently bonding to the halotriazine. Typical crosslinking agents include: ethyleneglycol dimethacrylate, diethyleneglycol bis-allyl carbonate.

A highly suitable and preferable lens material is hydroxyethylmethacrylate (HEMA) as disclosed in US Patent 2,976,576 and Re. 27,401. Two acceptable "hard" lens materials are cellulose acetate butyrate and those comprising polymethylmethacrylate (PMMA).

The method of preparing an UV radiation absorbing lens, preferably a contact lens, comprises the steps of contacting a lens made of polymeric lens material with a solution containing an effective amount of an ultraviolet radiation absorbing agent as disclosed in claim 1, and preferably a fluorotriazine ultraviolet radiation absorbing agent as disclosed in claim 1, capable of bonding with said lens material, e.g. and preferably by reacting with exoskeletal hydroxy, carboxy, amino, amido and/or mercapto groups of said polymeric lens material and removing said lens from said solution after a preselected period of time. Said process is preferably conducted in the presence of a base, and more preferably at a pH of at least 10. It is further preferred to maintain the solution for a preselected period of time to a temperature of at least 30°C.

A typical and recommended standard process for incorporating the reactive ultraviolet absorbing agent into the lens involves contacting the agent to the lens material, preferably under mild reaction conditions. For example, the lens is rinsed with deionized water and placed in a dry vial. Two milliliters each of a solution containing a reactive UV absorbing agent and diluted sodium carbonate solutions are then added to the vial. The vial containing the solutions and the lens is placed in a vial rack in a shaker bath at a set temperature and speed. After a set predetermined period of time has elapsed, the lens is removed from the vial, rinsed with deionized water, and extracted with a 10 % glycerine (aq) solution at 80°C for two hours. The lens is then rinsed with water and stored in a 0.9 % saline solution for 30 minutes. The transmission and/or absorbance spectrum of the lens can then be determined using a UV spectrophotometer.

It has also been found that the bonding of the ultraviolet absorbing agent and lens material may be enhanced by including an ammonium quaternary salt catalyst in the agent incorporating process. Said ammonium quaternary salts are preferably halogenides or sulfates, such as bromides, chlorides or hydrogen-sulfates of tetra-substituted ammonium-ions RₐR_{b}R_{c}R_{d}N wherein Rₐ, R_{b}, R_{c} and R_{d} are independently C₁-C₇alkyl, phenyl or phenyl-C₁-C₄-alkyl. Preferred are substituents such as C₁-C₄alkyl, e.g. butyl, ethyl or methyl, phenyl and phenyl-C₁-C₂alkyl, e.g. benzyl. Preferred combinations of substituents are tetra-C₁-C₇alkyl, phenyl-tris-C₁-C₇alkyl, phenyl-C₁-C₄-alkyl-tris-C₁-C₇alkyl and C₁-C₇alkyl-tris-phenyl-C₁-C₄alkyl. Examples of such ammonium quaternary salts include triethylbenzylammonium chloride, tetrabutylammonium hydrogen sulfate, phenyltrimethylammonium chloride, benzyltributylammonium chloride, tetrabutylammonium bromide, and tetramethylammonium chloride.

The following examples illustrate the instant invention. Temperatures are given in degree Centigrades.

### Example 1:

Cyanuric chloride, 18.4 g, is dissolved in 150 ml of warm acetone and the solution is poured into a stirred mixture of 200 g of ice and 200 ml of water. To this cyanuric chloride suspension is added simultaneously an aqueous solution made by dissolving 15.3 g of 4-amino salicylic acid in 120 ml of water containing 5.4 g of sodium carbonate and a dilute sodium carbonate solution (5.4 g in 50 ml of H₂O). After addition, the mixture is stirred at 5°-10°C for one and a half hour. The final pH of the mixture is 6.0.

The solid is collected by filtration, washed with water and air dried to obtain 30.6 g of 2,4-dichloro-6-[(3-hydroxy-4-carboxy)phenylamino]-s-triazine.

### Example 2:

To a mixture of 150 ml of acetone, 100 ml of water, 1 g of sodium carbonate and 6.5 g of dichloro-s-triazine, prepared as described in example 1 above, is added an aqueous solution (100 ml) of 7-amino-1,3-naphthalene disulfonic acid monopotassium salt (8.0 g) containing 1 g of sodium carbonate. The resulting mixture is refluxed for two hours. Most of acetone is then distilled off until the pot temperature reaches 80°C. The reaction mixture is cooled to about 10°C. The solid which forms is collected by filtration and dried to obtain 1.55 g of 2-chloro-4-[7-(1,3-disulfo)naphthylamino]-6-[(3-hydroxy-4-carboxy)phenylamino]-s-triazine and its sodium salts. More product (9.3 g) is obtained by adjusting the filtrate to pH = 3.0 and collecting the precipitate.

### Example 3:

Proceeding in a manner similar to that described in example 2 above, 9.0 g of 2,4-dichloro-6-[(3-hydroxy-4-carboxy)phenylamino]-s-triazine, 10.5 g of 3-amino-2,7-naphthalene disulfonic acid monosodium salt trihydrate and 3.0 g of sodium carbonate are interacted in water-acetone mixture to obtain 11.04 g of 2-chloro-4-[3-(2,7-disulfo)naphthylamino]-6-[(3-hydroxy-4-carboxy)phenylamino]-s-triazine and its sodium salts.

### Example 4:

Following the procedure described in example 1 above, 55.2 g of cyanuric chloride are interacted with 120 g of 7-amino-1,3-naphthalene disulfonic acid monopotassium salt in water-acetone mixture to obtain 81.6 g of 2,4-dichloro-6-[7-(1,3-disulfo)naphthylamino]-s-triazine.

### Example 5:

Proceeding in a manner similar to that described in example 2 above, 4.9 g of 2,4-dichloro-6-[7-(1,3-disulfo)naphthylamino]-s-triazine, 2.26 g of 2-(4-amino-2-hydroxyphenyl)benzotriazole and 2.1 g of sodium carbonate are interacted in water-acetone mixture to obtain 2-chloro-4-[(3-hydroxy-4-benzotriazo-2-yl)phenylamino]-6-[7-(1,3-disulfo)naphthylamino]-s-triazine and its sodium salts.

### Example 6:

Proceeding in a manner similar to that described in example 2 above, 47.4 g of 4-amino-2'-hydroxy-4'-methoxybenzophenone, 25.5 g of 2,4-dichloro-6-[7-(1,3-disulfo)naphthylamino]-s-triazine, and 10.5 g of sodium carbonate are interacted in water-acetone mixture to obtain 71.2 g of 2-chloro-4-[4-(2-hydroxy-4-methoxybenzoyl)phenylamino]-6-[7-(1,3-disulfo)naphthylamino]-s-triazine and its sodium salts.

### Example 7:

This example illustrates the effect of different catalysts on the incorporation of reactive UV absorbing agents in contact lenses: A series of corneal contact lenses is prepared and UV transmittance spectra are taken as set forth in the above-described standard process, except that 0.1 ml of an aqueous solution holding a catalyst are added to the vial containing the lens, a tri-sodium phosphate solution for maintaining a high pH and the aqueous solution having a UV blocking agent. The temperature of the bath is maintained at 45°C, the shaker bath speed is at 100 strokes per minute and the time the lenses remain in the shaker bath is two hours. A 1 % aqueous solution of the compound of example 6 is employed as the reactive UV blocking agent solution.

Transmittance data from UV absorbing lenses prepared as above using various catalyst solutions are compared to transmittance data from lenses identically prepared except that no catalyst is employed. A sharp decrease in the transmittance curve for lenses prepared without a catalyst is found to occur around 360 nm, and transmittance spectra for these lenses exhibit a shoulder in the region from 275 to 360 nm with a small peak occurring around 290 nm. As is shown in Table 1, the quaternary ammonium salt catalysts substantially improve the UV absorbing characteristics of the lenses.

**Table 1**

| Catalyst | % T at 290 nm | Transmittance characteristics in the 275-360 nm range |
|---|---|---|
| 1. no catalyst | 2.3 % | shoulder |
| 2. 10 % Tyloxapol (aq) | 6.9 % | pronounced shoulder |
| 3. 10 % Varsulf SBFA-30 (aq) | 4.6 % | distinct shoulder |
| 4. 10 % Pluronic E-127 (aq) | 2.3 % | similar to no catalyst |
| 5. 5 % triethylbenszylammonium chloride (aq) | <1 % | no shoulder |
| 6. 5 % cetylpyridinium chloride (aq) | 9.2 % | very prominent shoulder |
| 7. 5 % tetrabutylammonium hydrogen sulfate (aq) | <1 % | no shoulder |
| 8. 5 % p-dimethylaminopyridine | 2.3 % | similar to no catalyst |

### Example 8:

This example further illustrates the effectiveness of different quaternary ammonium salts on the incorporation of absorbing agents in contact lenses: A series of corneal contact lenses is prepared and UV transmittance and absorbance spectra are taken as set forth in example 7, except that 0.2 ml of a 5 % aqueous solution of a quaternary ammonium salt is added to the vial containing the lens, the tri-sodium phosphate solution, and the solution containing a UV blocking agent. The temperature of the bath is maintained at 45°C, the shaker bath speed is 110 strokes per minute, and the time the lenses remain in the shaker bath is two hours. A 1 % aqueous solution of the compound of example 6 is employed as the reactive UV blocking agent solution. Five percent (5 %) aqueous solutions of (1) tetrabutylammonium hydrogen sulfate, (2) phenyltrimethylammonium chloride, (3) benzyltributylammonium chloride, (4) tetrabutylammonium bromide, (5) tetramethylammonium chloride and (6) a polyquat solution are tested in this example.

Transmittance data from UV absorbing lenses prepared utilizing (1), (2), (3), and (4) show the superior UV absorbing characteristics of these lenses compared to lenses prepared without any catalyst. The transmittance peak at around 290 nm that appears in a lens prepared without any catalyst and the shoulder in the 275 to 360 nm region are not present in lenses prepared in the presence of (1), (2), (3) or (4). Absorbance of UV radiation in the 290 nm to 400 nm region is greatest for lenses prepared using (3) followed by those prepared using (4), (1) and (2) respectively. The use of (5) as a catalyst produces lenses with UV absorbing characteristics only slightly better than lenses prepared in the absence of a catalyst. However, the use of (6) as a catalyst retards the incorporation of the UV absorbing agent in the lens, and lenses prepared in the presence of (6) show poor UV absorption in the 260 to 400 nm region.

### Example 9:

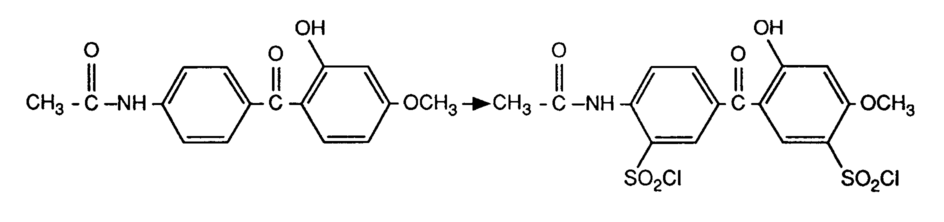

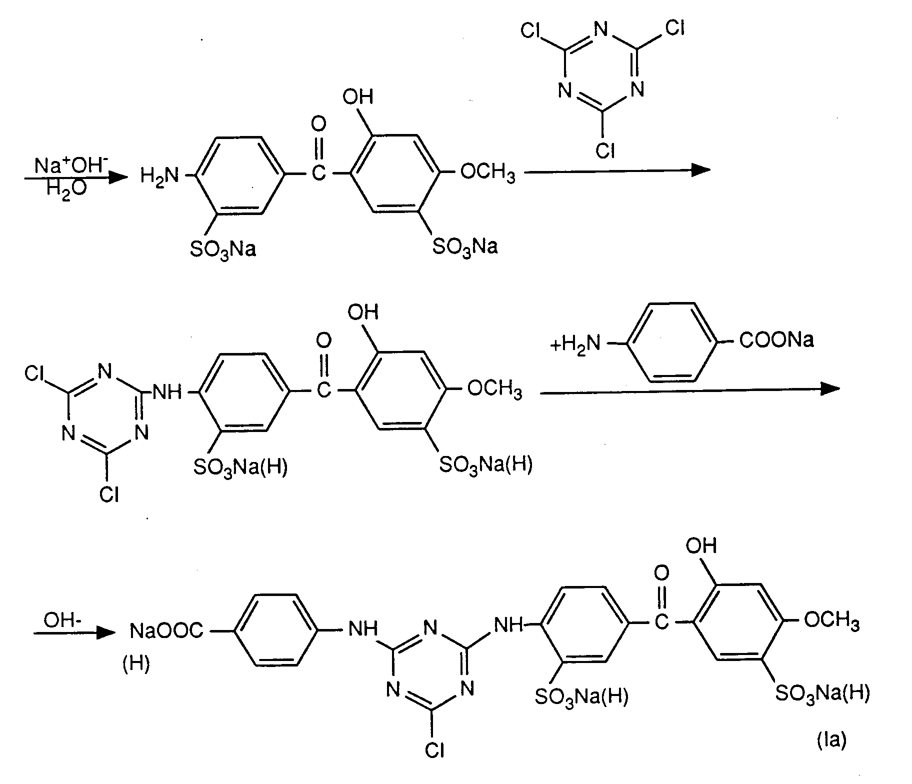

A. 100 ml of chlorosulfonic acid are charged into a 500 ml flask and cooled to 5°C. Then, 20 g of 4′-acetamido-2-hydroxy-4-methoxybenzophenone are added to the flask over a period of 15 minutes. The reaction mixture is maintained at a temperature below 20°C during the addition. After the addition, the reaction mixture is stirred at room temperature for 16 hours. The reaction mixture is added dropwise into 1.5 liters of ice water and a precipitate is formed. The precipitate is collected and washed two times with 50 ml of ice water, then air dried. The precipitate is placed in 120 ml of water, and dilute NaOH is slowly added until the solid dissolves. The solution has a pH of 12 and is kept at 90°C for 30 minutes, then cooled to room temperature.
B. A cyanuric chloride suspension is prepared by dissolving 12 g of cyanuric chloride in 50 ml of warm acetone, and quickly dispersed into 150 ml of ice water. At 5°C, the solution from part A is quickly added to the cyanuric chloride suspension. The reaction is stirred at approximately 10°C for three hours whereby a gel material is formed. Water is added to dilute the gel material until the total volume is 400 ml and the pH is 3.0.
C. A mixture of 100 ml of the gel material solution from part B, 2.5 g of p-aminobenzoic acid which is predissolved in dilute base, 3.0 g of sodium carbonate and 100 ml of water is heated at 90°C for three hours. The mixture is then cooled to room temperature and the pH is adjusted from 9.2 to 7.0 with 3N·HCl. The solution is then evaporated to dryness and the residue is extracted with hot acetone several times to obtain 2-chloro-4-[(4-carboxy)phenylamino]-6-{2-sulfo-4-[(2-hydroxy-4-methoxy-5-sulfo)benzoyl]phenylamino)-s-triazine (compound Ia) and/or its sodium salts as a yellow solid, which is an ultraviolet radiation absorbing agent.

### Example 10:

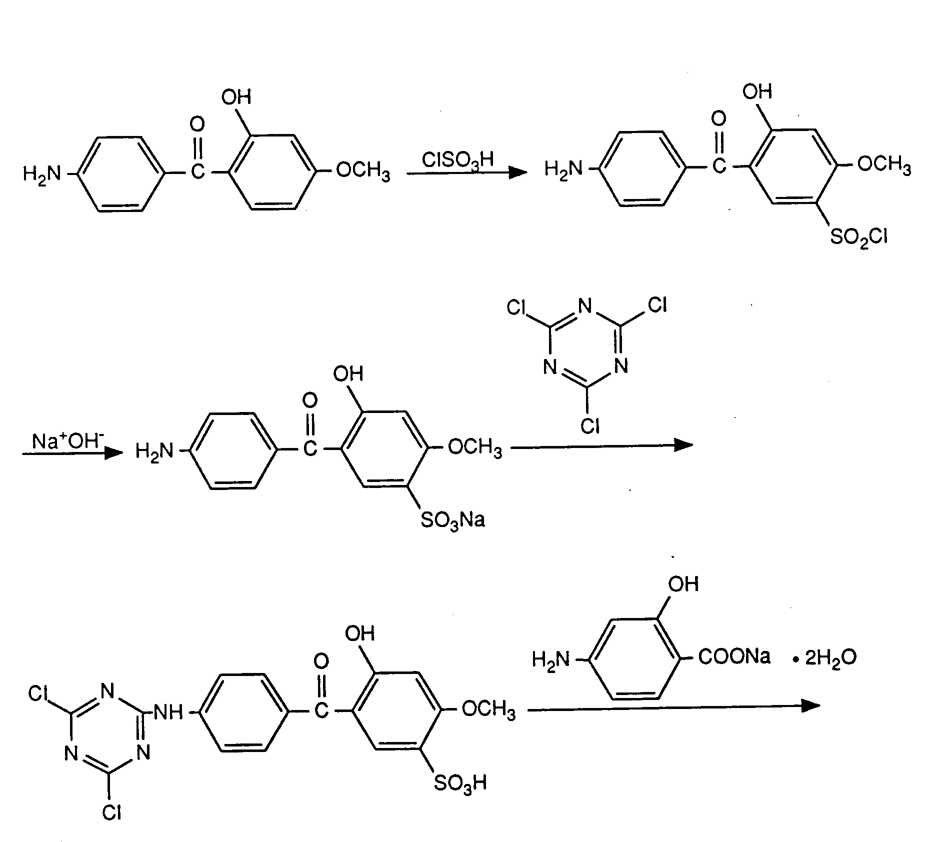

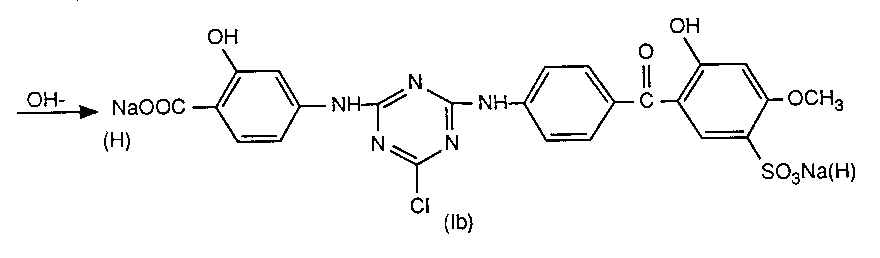

A. 100 ml of chlorosulfonic acid are charged into a flask and cooled at approximately 5°C in an ice bath. 20 g of 4′-amino-2-hydroxy-4-methoxybenzophenone are slowly added over a period of 20 minutes. The addition is performed at a temperature below 20°C. After addition, the reaction mixture is stirred at room temperature for four hours. The mixture is then quenched dropwise into 1.5 liters of ice water and a precipitate is formed. The precipitate is collected, washed two times with 50 ml of ice water, and air dried to obtain 28.5 g. 28.3 g of the precipitate are suspended in 150 ml of water. 25 % NaOH is slowly added to dissolve the precipitate and the pH of the solution is adjusted to 11.5 and is kept at 90°C for thirty minutes, then cooled to room temperature. The resulting orange solution has a total volume of 300 ml.
B. A cyanuric chloride dispersion is prepared by dissolving 15 g of cyanuric chloride in 70 ml of warm acetone, and quickly dispersed into 200 ml of ice water. The beaker which has been holding the cyanuric chloride is rinsed with an additional 30 ml of acetone, which is then added to the dispersion. The dispersion is cooled to 5°C.

The orange solution from part (A) is quickly added into the cyanuric chloride dispersion and the resulting mixture is stirred at between approximately 5° and 10°C for three hours. The pH of the mixture is 2.7. A small amount of water is added, and the final volume is adjusted to 900 ml.
C. 225 ml of the mixture from (B), 4.2 g of 4-aminosalicylic acid sodium salt dihydrate, 3.0 g of Na₂CO₃ and 150 ml of water are mixed. The pH is adjusted to 9.5 by adding NaOH, and the reaction mixture is heated at 90°C for three hours, then cooled to room temperature. The pH is then adjusted to 7.0 with 3N·HCl. The mixture is evaporated to dryness. The residue is extracted with hot acetone several times to get 17.79 g of 2-chloro-4-[(3-hydroxy-4-carboxy)phenylamino]-6-{4-[(2-hydroxy-4-methoxy-5-sulfo)benzoyl]phenylamino}-s-triazine (Compound Ib) and its sodium salts, as a yellow solid, which is a UV absorbing agent.

### Example 11:

Proceeding in a manner similar to that described in example 9, part C above, except that 4-amino salicylic acid is substituted for p-aminobenzoic acid, results in the formation of 2-chloro-4-[(3-hydroxy-4-carboxy)phenylamino]-6-{2-sulfo-4-[(2-hydroxy-4-methoxy-5-sulfo)benzoyl]phenylamino}-s-triazine and its sodium salts.

### Example 12:

Proceeding in a manner similar to that described in example 10, part C above, except that p-aminobenzoic acid is substituted for 4-aminosalicylic acid to obtain 2-chloro-4-[(4-carboxy)phenylamino]-6-{4-[(2-hydroxy-4-methoxy-5-sulfo)benzoyl]phenylamino}-s-triazine and its sodium salts.

### Example 13:

A typical process for applying the absorbing agent to the lens is now set forth. A mixture of 2 ml of 0.05 to 5.0 % (aq) stock solution of ultraviolet radiation absorbing agent, 2 ml of 5 to 10 % (aq) Na₃PO₄·12H₂O, and 0.2 ml of 1 to 10 % (aq) solution of tetrabutylammonium bromide is prepared and heated at 50°C for 60 minutes with agitation. A clear lens comprised of hydroxyethyl methacrylate (HEMA) is then soaked in the mixture until the agent bonds to the lens. The lens is then neutralized with a buffered saline solution (pH = 7.0), after which the lens is extracted with 10 % glycerine in an extraction bath until there is no UV absorbing agent leaching out. This is determined by a UV spectrophotometer. After the extraction process, the lens is boiled in distilled water, and then buffered with saline to remove any remaining glycerine.

## Claims

1. An ultraviolet radiation absorbing agent of the formula I 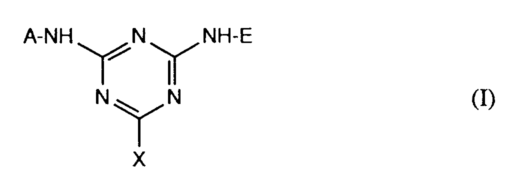 wherein A is an ultraviolet radiation absorbing group; E is an aqueous soluble group E₁ and X is Cl or F according to formula (IB), or E is an ultraviolet radiation absorbing group E₂ and X is Cl according to formula (IA); 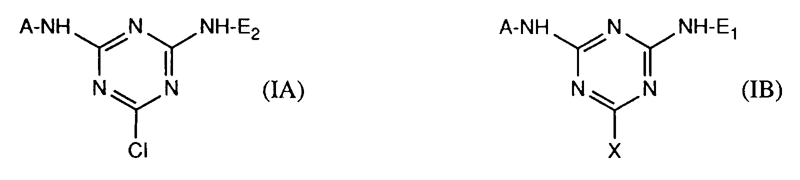 wherein A and E₂ are either identical to or dissimilar to each other and wherein A and E₂ are selected from the radicals of benzoic acid, salicylic acid, substituted benzophenone and substituted benzotriazoles, benzoic acid esters, acrylates, oxalic acid diamides, and hydroxy phenyltriazines; and
wherein E₁ is an aqueous soluble group represented by the radical of the formula 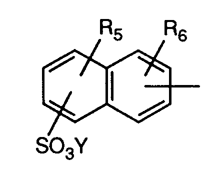 wherein Y is hydrogen, R₅ and R₆ are hydrogen, C₁-C₁₈alkyl, C₁-C₁₈alkoxy, halogen, nitro, hydroxy, carboxy or sulfo and salts of said radicals;
with the proviso that A and E₂ are not simultaneously selected from 2-(o-hydroxyaryl)benzotriazole.

2. The agents of claim 1 wherein A and E₂ is selected from the group of radicals consisting of 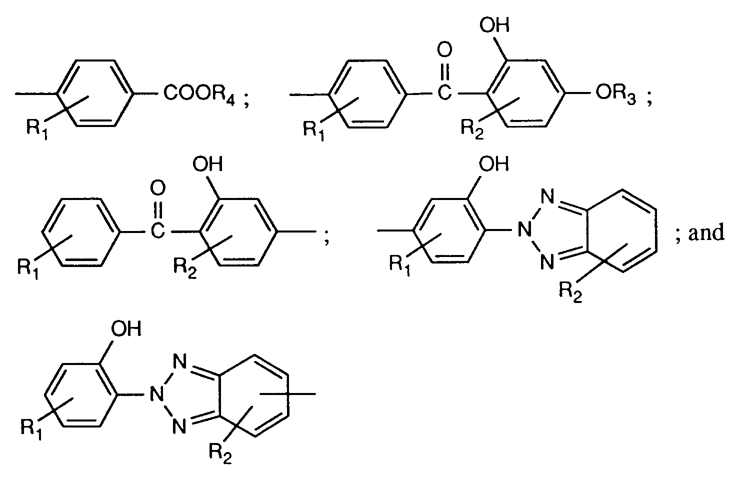 wherein R₁ and R₂ are independently of each other hydrogen, C₁-C₁₈alkyl, C₁-C₁₈alkoxy, halogen, nitro, hydroxy, carboxy or sulfo (SO₃H); and R₃ and R₄ are hydrogen or C₁-C₁₈alkyl; and salts thereof.

3. The agent of claim 2, wherein said C₁-C₁₈alkyl is C₁-C₄alkyl, and C₁C₁₈alkoxy is C₁-C₄alkoxy.

4. The ultraviolet radiation absorbing agent of claim 1 which is selected from 2-chloro-4-[(4-carboxy)phenylamino]-6-{2-sulfo-4-[(2-hydroxy-4-methoxy-5-sulfo)benzoyl]phenylamino}-s-triazine; 2-chloro-4-[(3-hydroxy-4-carboxy)phenylamino]-6-{4-[(2-hydroxy-4-methoxy-5-sulfo)benzoyl]phenylamino}-s-triazine; 2-chloro-4-[(3-hydroxy-4-carboxy)phenylamino]-6-{2-sulfo-4-[(2-hydroxy-4-methoxy-5-sulfo)benzoyl]phenylamino}-s-triazine; 2-chloro-4-[(4-carboxy)phenylamino]-6-{4-[(2-hydroxy-4-methoxy-5-sulfo)benzoyl]phenylamino}-s-triazine; and sodium salts thereof.

5. The agent of claim 1, wherein in the radical E₁ said C₁-C₁₈alkyl and C₁-C₁₈alkoxy are selected form C₁-C₄alkyl and C₁-C₄alkoxy.

6. The agent of claim 1, which is selected from 2-chloro-4-[7-(1,3-disulfo)naphthylamino]-6-[(3-hydroxy-4-carboxy)phenylamino]-s-triazine; 2-chloro-4-[3-(2,7-disulfo)naphthylamino]-6-[(3-hydroxy-4-carboxy)phenylamino]-s-triazine; 2-chloro-4-[(3-hydroxy-4-benzotriazo-2-yl)phenylamino]-6-[7-(1,3-disulfo)naphthylamino]-s-triazine; 2-chloro-4-[4-(2-hydroxy-4-methoxybenzoyl)phenylamino]-6-[7-(1,3-disulfo)naphthylamino]-s-triazine, and sodium salts thereof.

7. The agent of claim 1 wherein A and E₂ are different groups.

8. The agent of claim 1 wherein A and E₂ are identical groups.

9. An ultraviolet radiation absorbing lens of a polymeric lens material characterized in that an effective UV radiation absorbing amount of an ultraviolet radiation absorbing agent of formula I according to the disclosure of claim 1 is covalently bonded to the polymeric lens material.

10. The ultraviolet radiation absorbing lens of claim 9, wherein said polymeric lens material has at least one functional group available for reacting with the halotriazine of formula I selected from the group consisting of hydroxyl, carboxyl, amino, amido, and mercapto, and mixtures thereof.

11. The ultraviolet radiation absorbing lens of claim 10, wherein said polymeric lens material comprises hydroxyethyl methacrylate.

12. The ultraviolet radiation absorbing lens of claim 10, wherein said polymeric lens material comprises cellulose acetate butyrate or polymethylmethacrylate.

13. The ultraviolet radiation absorbing lens of claim 9 which is a contact lens.

14. The ultraviolet radiation absorbing lens of claim 9 which is an intraocular lens.

15. A method of preparing an ultraviolet radiation absorbing lens, comprising the steps of contacting a lens made of polymeric lens material with a solution containing an effective amount of an ultraviolet radiation absorbing agent of formula I as defined in claim 1, capable of bonding with said lens material and removing said lens from said solution after a preselected period of time.

16. A process for preparing an ultraviolet radiation absorbing contact lens material, comprising the steps of preparing a solution of effective amounts of a halotriazine reactive ultraviolet radiation absorbing agent of formula I as defined in claim 1 and a base; maintaining said solution for a preselected period of time at a temperature up to at least 30°C; adding a polymeric lens material to said solution; and removing said polymeric lens material from the solution after a preselected time.

17. A process for preparing an ultraviolet radiation absorbing contact lens material, comprising the steps of preparing a solution of effective amounts of the halotriazine reactive ultraviolet radiation absorbing agent of formula I as defined in claim 1 and a base; maintaining said solution for a preselected period of time at a temperature up to at least 30°C; adding a polymeric lens material to said solution; and removing said polymeric lens material from the solution after a preselected time.

18. A process according to claim 16, and further comprising the step of adding an effective amount of an ammonium quaternary salt to said solution prior to said maintaining step.

19. A process according to claim 18 wherein the quaternary ammonium salt is a halogenide or sulfate of tetra-substituted ammonium-ions RₐR_{b}R_{c}R_{d}N wherein Rₐ, R_{b}, R_{c} and R_{d} are independently C₁-C₇alkyl, phenyl or phenyl-C₁-C₄-alkyl.

20. A process according to claim 18, wherein said ammonium quaternary salt is selected from the group consisting of triethylbenzylammonium chloride, tetrabutylammonium hydrogen sulfate, phenyltrimethylammonium chloride, benzyltributylammonium chloride, tetrabutylammonium bromide, and tetramethylammonium chloride.

21. A process according to claim 16, and further including the steps of shaping said polymeric material into a contact lens.

22. A process according to claim 16, wherein said solution has a pH of at least 10.

23. A process according to claim 16, wherein said absorbing agent is a fluorotriazine reactive ultraviolet radiation absorbing agent.

24. Use of an ultraviolet radiation absorbing agent of formula I as defined in claim 1 for preparing an ultraviolet radiation absorbing contact lens.

25. Use of an ultraviolet radiation absorbing agent of formula I as defined in claim 1 for preparing an ultraviolet radiation absorbing intraocular lens.

## Patentansprüche

1. Ultraviolettstrahlung-absorbierendes Mittel der Formel I 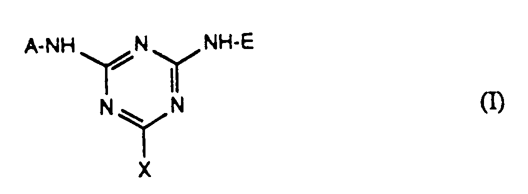 worin A eine Ultraviolettstrahlung-absorbierende Gruppe darstellt, E eine in Wasser lösliche Gruppe E₁ darstellt und X Cl oder F nach Formel (IB) darstellt oder E eine Ultraviolettstrahlung-absorbierende Gruppe E₂ darstellt und X Cl nach Formel (IA) darstellt; 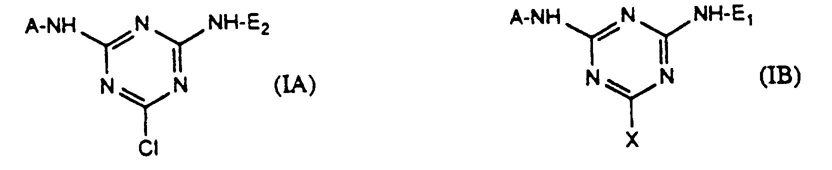 worin A und E₂ entweder gleich sind oder voneinander verschieden sind und worin A und E₂ ausgewählt sind aus den Resten von Benzoesäure, Salicylsäure, substituiertem Benzophenon und substituierten Benzotriazolen, Benzoesäureestern, Acrylaten, Oxalsäurediamiden und Hydroxyphenyltriazinen und
worin E₁ eine in Wasser lösliche Gruppe darstellt, wiedergegeben durch den Rest mit der Formel 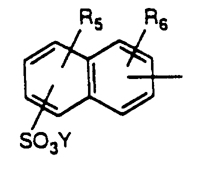 worin Y Wasserstoff darstellt, R₅ und R₆ Wasserstoff, C₁-C₁₈-Alkyl, C₁-C₁₈-Alkoxy, Halogen, Nitro, Hydroxy, Carboxy oder Sulfo darstellen, und Salze der Reste;
mit der Maßgabe, daß A und E₂ nicht gleichzeitig ausgewählt sind aus 2-(o-Hydroxyaryl)benzotriazol.

2. Mittel nach Anspruch 1, worin A und E₂ ausgewählt sind aus der Gruppe von Resten, bestehend aus 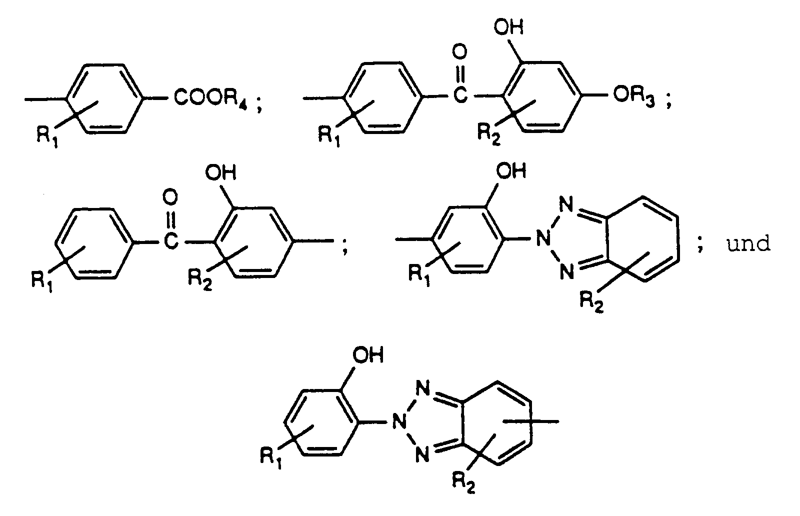 worin R₁ und R₂ unabhängig voneinander Wasserstoff, C₁-C₁₈-Alkyl, C₁-C₁₈-Alkoxy, Halogen, Nitro, Hydroxy, Carboxy oder Sulfo (SO₃H) darstellen und R₃ und R₄ Wasserstoff oder C₁-C₁₈-Alkyl darstellen und Salze davon.

3. Mittel nach Anspruch 2, wobei die C₁-C₁₈-Alkylgruppe C₁-C₄-Alkyl darstellt und die C₁-C₁₈-Alkoxygruppe C₁-C₄-Alkoxy darstellt.

4. Ultraviolettstrahlung-absorbierendes Mittel nach Anspruch 1, das ausgewählt ist aus 2-Chlor-4-[(4-carboxy)phenylamino]-6-{2-sulfo-4-[(2-hydroxy-4-methoxy-5-sulfo)benzoyl]phenylamino}-s-triazin, 2-Chlor-4-[(3-hydroxy-4-carboxy)phenylamino]-6-{4-[(2-hydroxy-4-methoxy-5-sulfo)benzoyl]phenylamino}-s-triazin, 2-Chlor-4-[(3-hydroxy-4-carboxy)-phenylamino]-6-{2-sulfo-4-[(2-hydroxy-4-methoxy-5-sulfo)benzoyl[phenylamino}-s-triazin, 2-Chlor-4-[(4-carboxy)phenylamino]-6-{4-[(2-hydroxy-4-methoxy-5-sulfo)benzoyl]phenylamino}-s-triazin und Natriumsalzen davon.

5. Mittel nach Anspruch 1, wobei in dem Rest E₁ die C₁-C₁₈-Alkylgruppe und die C₁-C₁₈-Alkoxygruppe aus C₁-C₄-Alkyl und C₁-C₄-Alkoxy ausgewählt sind.

6. Mittel nach Anspruch 1, das ausgewählt ist aus 2-Chlor-4-[7-(1,3-disulfo)naphthylamino]-6-[(3-hydroxy-4-carboxy)phenylamino]-s-triazin, 2-Chlor-4-[3-(2,7-disulfo)naphthylamino]-6-[(3-hydroxy-4-carboxy)phenylamino]-s-triazin, 2-Chlor-4-[(3-hydroxy-4-benzotriazo-2-yl)phenylamino]-6-[7-(1,3-disulfo)naphthylamino]-s-triazin, 2-Chlor-4-[4-(2-hydroxy-4-methoxybenzoyl)phenylamino]-6-[7-(1,3-disulfo)naphthylamino]-s-triazin und Natriumsalzen davon.

7. Mittel nach Anspruch 1, worin A und E₂ unterschiedliche Gruppen sind.

8. Mittel nach Anspruch 1, worin A und E₂ gleiche Gruppen sind.

9. Ultraviolettstrahlung-absorbierende Linse aus einem polymeren Linsenmaterial, dadurch gekennzeichnet, daß eine wirksame UV-Strahlung-absorbierende Menge eines Ultraviolettstrahlung-absorbierenden Mittels der Formel I nach der Offenbarung von Anspruch 1 kovalent an das polymere Linsenmaterial gebunden ist.

10. Ultraviolettstrahlung-absorbierende Linse nach Anspruch 9, wobei das polymere Linsenmaterial mindestens eine funktionelle Gruppe aufweist, die zum Umsetzen mit dem Halogentriazin der Formel I zur Verfügung steht, ausgewählt ist aus der Gruppe, bestehend aus Hydroxyl, Carboxyl, Amino, Amido und Mercapto und Gemischen davon.

11. Ultraviolettstrahlung-absorbierende Linse nach Anspruch 10, wobei das polymere Linsenmaterial Hydroxyethylmethacrylat umfaßt.

12. Ultraviolettstrahlung-absorbierende Linse nach Anspruch 10, wobei das polymere Linsenmaterial Celluloseacetatbutyrat oder Polymethylmethacrylat umfaßt.

13. Ultraviolettstrahlung-absorbierende Linse nach Anspruch 9, die eine Kontaktlinse darstellt.

14. Ultraviolettstrahlung-absorbierende Linse nach Anspruch 9, die eine intraokulare Linse darstellt.

15. Verfahren zur Herstellung einer Ultraviolettstrahlung-absorbierenden Linse, umfassend die Schritte von Inkontaktbringen einer aus polymerem Linsenmaterial hergestellten Linse mit einer Lösung, die eine wirksame Menge eines wie in Anspruch 1 definierten Ultraviolettstrahlung-absorbierenden Mittels der Formel I enthält, das in der Lage ist, an das Linsenmaterial zu binden und Entfernen der Linse aus der Lösung nach einem vorher ausgewählten Zeitraum.

16. Verfahren zur Herstellung eines Ultraviolettstrahlung-absorbierenden Kontaktlinsenmaterials, umfassend die Schritte der Herstellung einer Lösung von wirksamen Mengen eines Halogentriazin-reaktiven, Ultraviolettstrahlung-absorbierenden Mittels der wie in Anspruch 1 definierten Formel I, und einer Base, Halten der Lösung für einen vorher ausgewählten Zeitraum bei einer Temperatur bis zu mindestens 30°C, Zugabe eines polymeren Linsenmaterials zu der Lösung und Entfernen des polymeren Linsenmaterials aus der Lösung nach einem vorher ausgewählten Zeitraum.

17. Verfahren zur Herstellung eines Ultraviolettstrahlung-absorbierenden Kontaktlinsenmaterials, umfassend die Schritte der Herstellung einer Lösung von wirksamen Mengen des Halogentriazin-reaktiven Ultraviolettstrahlung-absorbierenden Mittels der Formel I nach Anspruch 1 und einer Base, Halten der Lösung für einen vorher ausgewählten Zeitraum, bei einer Temperatur bis zu mindestens 30°C, Zugabe eines polymeren Linsenmaterials zu der Lösung und Entfernen des polymeren Linsenmaterials aus der Lösung nach einem vorher ausgewählten Zeitraum.

18. Verfahren nach Anspruch 16, das weiterhin den Schritt der Zugabe einer wirksamen Menge eines quaternären Ammoniumsalzes zu der Lösung vor dem Halteschritt umfaßt.

19. Verfahren nach Anspruch 18, wobei das quaternäre Ammoniumsalz ein Halogenid oder Sulfat von tetra-substituierten Ammoniumionen RₐR_{b}R_{c}R_{d}N darstellt, worin Rₐ, R_{b}, R_{c} und R_{d} unabhängig C₁-C₇-Alkyl, Phenyl oder Phenyl-C₁-C₄-alkyl darstellen.

20. Verfahren nach Anspruch 18, wobei das quaternäre Ammoniumsalz ausgewählt ist aus der Gruppe, bestehend aus Triethylbenzylammoniumchlorid, Tetrabutylammoniumhydrogensulfat, Phenyltrimethylammoniumchlorid, Benzyltributylammoniumchlorid, Tetrabutylammoniumbromid und Tetramethylammoniumchlorid.

21. Verfahren nach Anspruch 16, das weiterhin die Schritte des Formens des polymeren Materials zu einer Kontaktlinse einschließt.

22. Verfahren nach Anspruch 16, wobei die Lösung einen pH-Wert von mindestens 10 aufweist.

23. Verfahren nach Anspruch 16, wobei das absorbierende Mittel ein Fluortriazin-reaktives Ultraviolettstrahlung-absorbierendes Mittel darstellt.

24. Verwendung eines Ultraviolettstrahlung-absorbierenden Mittels der wie in Anspruch 1 definierten Formel I zum Herstellen einer Ultraviolettstrahlung-absorbierenden Kontaktlinse.

25. Verwendung eines Ultraviolettstrahlung-absorbierenden Mittels der wie in Anspruch 1 definierten Formel I zum Herstellen einer intraokularen, Ultraviolettstrahlung-absorbierenden Linse.

## Revendications

1. Un agent absorbant le rayonnement ultraviolet de formule I 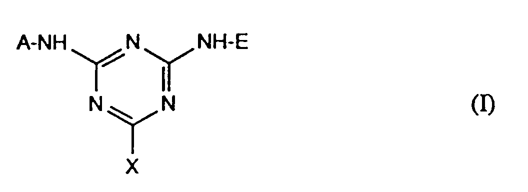 où A signifie un groupe absorbant le rayonnement ultraviolet; E signifie un groupe aqueux soluble E₁ et X signifie Cl ou F selon la formule (IB), ou E signifie un groupe absorbant le rayonnement ultraviolet E₂ et X signifie Cl selon la formule (IA); 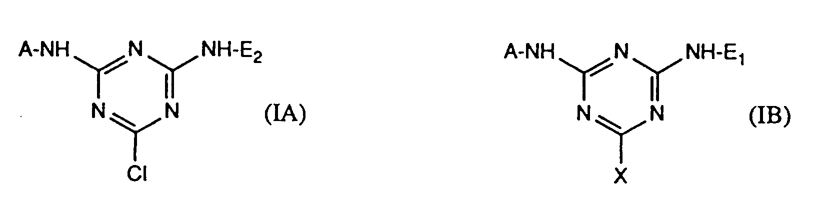 où A et E₂ sont soit identiques ou différents l'un de l'autre et où A et E₂ sont choisis parmi les restes de l' acide benzoïque, de l'acide salicylique, de la benzophénone substituée et des benzotriazoles substitués, des esters de l'acide benzoïque, des acrylates, des diamides de l'acide oxalique, et des hydroxy-phényltriazines; et
où E₁ signifie un groupe aqueux soluble représenté par le groupe de formule 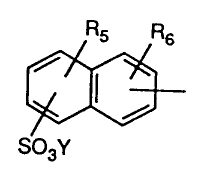 où Y signifie l'hydrogène, R₅ et R₆ signifient l'hydrogène, un groupe C₁-C₁₈alkyle, C₁-C₁₈alcoxy, un halogène, nitro, hydroxy, carboxy ou sulfo et les sels desdits groupes;
A et E₂ ne devant pas être choisis simultanément parmi un groupe 2-(o-hydroxyaryl)benzotriazole.

2. Les agents selon la revendication 1 où A et E₂ sont choisis parmi le groupe comprenant 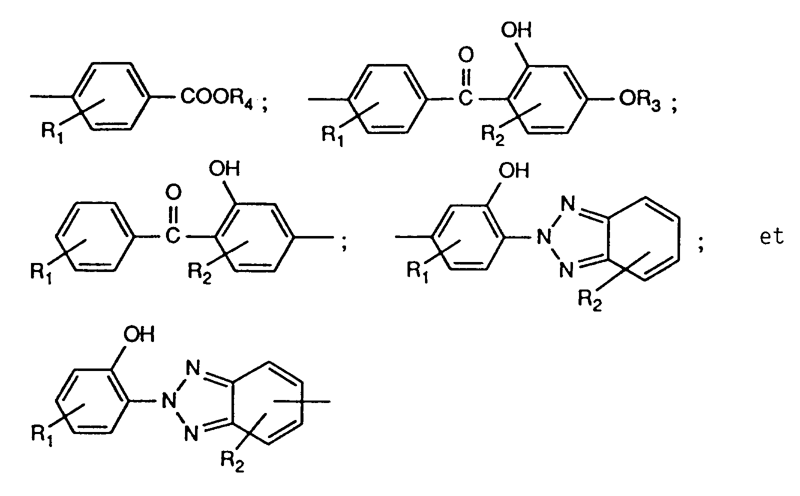 où R₁ et R₂ signifient indépendamment l'un de l'autre, l'hydrogène, un groupe C₁-C₁₈alkyle, C₁-C₁₈alcoxy, un halogène, nitro, hydroxy, carboxy ou sulfo (SO₃H); et R₃ et R₄ signifient l'hydrogène ou un groupe C₁-C₁₈alkyle; et leurs sels.

3. L'agent selon la revendication 2, où ledit groupe C₁-C₁₈alkyle signifie un groupe C₁-C₄alkyle et le groupe C₁-C₁₈alcoxy signifie un groupe C₁-C₄alcoxy.

4. L'agent absorbant le rayonnement ultraviolet selon la revendication 1 qui est choisi parmi la 2-chloro-4-[(4-carboxy)phénylamino]-6-{2-sulfo-4-[(2-hydroxy-4-méthoxy-5-sulfo)benzoyl]phénylamino}-s-triazine; la 2-chloro-4-[(3-hydroxy-4-carboxy)phénylamino]-6-{4-[(2-hydroxy-4-méthoxy-5-sulfo)benzoyl]phénylamino}-s-triazine; la 2-chloro-4-[(3-hydroxy-4-carboxy)phénylamino]-6-{2-sulfo-4-[(2-hydroxy-4-méthoxy-5-sulfo)benzoyl]phénylamino}-s-triazine; la 2-chloro-4-[(4-carboxy)phénylamino]-6-{4-[(2-hydroxy-4-méthoxy-5-sulfo)benzoyl]phénylamino}-s-triazine; et ses sels de sodium.

5. L'agent selon la revendication 1 où, dans le groupe E_{1,} lesdits groupes C₁-C₁₈alkyle et C₁-C₁₈alcoxy sont choisis parmi un groupe C₁-C₄alkyle et C₁-C₄alcoxy.

6. L'agent selon la revendication 1 qui est choisi parmi la 2-chloro-4-[(7-(1,3-disulfo)naphtylamino]-6-[(3-hydroxy-4-carboxy)phénylamino]-s-triazine; la 2-chloro-4-[3-(2,7-disulfo)-naphtylamino]-6-[(3-hydroxy-4-carboxy)phénylamino]-s-triazine; la 2-chloro-4-[(3-hydroxy-4-benzotriazo-2-yl) phénylamino]-6-[7-(1,3-disulfo)naphtylamino]-s-triazine; la 2-chloro-4-[4-(2-hydroxy-4-méthoxybenzoyl) phénylamino]-6-[7-(1,3-disulfo)naphtylamino]-s-triazine, et ses sels de sodium.

7. L'agent selon la revendication 1 où A et E₂ signifient des groupes différents.

8. L'agent selon la revendication 1 où A et E₂ signifient des groupes identiques.

9. Une lentille absorbant le rayonnement ultraviolet en une matière polymère pour lentilles caractérisée en ce qu'une quantité efficace pour absorber le rayonnement ultraviolet d'un agent absorbant le rayonnement ultraviolet de formule I selon la revendication 1 est liée par covalence à la matière polymère pour lentilles.

10. La lentille absorbant le rayonnement ultraviolet selon la revendication 9, où ladite matière polymère pour lentilles a au moins un groupe fonctionnel disponible pour la réaction avec la halotriazine de formule I choisie parmi le groupe comprenant un groupe hydroxy, carboxy, amino, amido et mercapto, et leurs mélanges.

11. La lentille absorbant le rayonnement ultraviolet selon la revendication 10, où ladite matière polymère pour lentilles comprend du méthacrylate d'hydroxyéthyle.

12. La lentille absorbant le rayonnement ultraviolet selon la revendication 10, où ladite matière polymère pour lentilles comprend de l'acéto-butyrate de cellulose ou du méthacrylate de polyméthyle.

13. La lentille absorbant le rayonnement ultraviolet selon la revendication 9 qui est une lentille de contact.

14. La lentille absorbant le rayonnement ultraviolet selon la revendication 9 qui est une lentille intraoculaire.

15. Une méthode de préparation d'une lentille absorbant le rayonnement ultraviolet, comprenant les étapes de mise en contact d'une lentille faite en une matière polymère pour lentilles avec une solution contenant une quantité efficace d'un agent absorbant le rayonnement ultraviolet de formule I tel que défini à la revendication 1, capable de se lier avec ladite matière pour lentilles et le retrait de ladite lentille de ladite solution après une période de temps pré-déterminée.

16. Un procédé de préparation d'une matière pour lentilles de contact absorbant le rayonnement ultraviolet, comprenant les étapes de préparation d'une solution comprenant des quantités efficaces d'un agent absorbant le rayonnement ultraviolet réactif, à base d'une halotriazine de formule I tel que défini à la revendication 1 et d'une base; le maintien de ladite solution pendant une période de temps pré-déterminée à une température maximale d'au moins 30°C; l'addition d'une matière polymère pour lentilles à ladite solution; et le retrait de ladite matière polymère pour lentilles de la solution après un temps pré-déterminé.

17. Un procédé de préparation d'une matière pour lentilles de contact absorbant le rayonnement ultraviolet, comprenant les étapes de préparation d'une solution comprenant des quantités efficaces d'un agent absorbant le rayonnement ultraviolet réactif, à base d'une halotriazine de formule I tel que défini à la revendication 1 et d'une base; le maintient de ladite solution pendant une période de temps pré-déterminée à une température maximale d'au moins 30°C; l'addition d'une matière polymère pour lentilles à ladite solution; et le retrait de ladite matière polymère pour lentilles de la solution après un temps pré-déterminé.

18. Un procédé selon la revendication 16, comprenant également l'étape d'addition d'une quantité efficace d'un sel d'ammonium quaternaire à ladite solution avant ladite étape de maintien.

19. Un procédé selon la revendication 18 où le sel d'ammonium quaternaire est un halogénure ou un sulfate d'ions ammonium tétrasubstitués RₐR_{b}R_{c}R_{d}N où Rₐ, R_{b}, R_{c}, et R_{d} signifient indépendamment un groupe C₁-C₇alkyle, phényle ou phényl-C₁-C₄-alkyle.

20. Un procédé selon la revendication 18, où ledit sel d'ammonium quaternaire est choisi parmi le groupe comprenant le chlorure de triéthylbenzylammonium, l'hydrogéno-sulfate de tétrabutylammonium, le chlorure de phényltriméthylammonium, le chlorure de benzyltributylammonium, le bromure de tétrabutylammonium et le chlorure de tétraméthylammonium.

21. Un procédé selon la revendication 16, comprenant également les étapes de façonnage de ladite matière polymère en une lentille de contact.

22. Un procédé selon la revendication 16, où ladite solution a un pH d'au moins 10.

23. Un procédé selon la revendication 16, où ledit agent absorbant est un agent absorbant le rayonnement ultraviolet réactif, à base d'une fluorotriazine.

24. L'utilisation d'un agent absorbant le rayonnement ultraviolet de formule I tel que défini à la revendication 1, pour la préparation d'une lentille de contact absorbant le rayonnement ultraviolet.

25. L'utilisation d'un agent absorbant le rayonnement ultraviolet de formule I tel que défini à la revendication 1, pour la préparation d'une lentille intraoculaire absorbant le rayonnement ultraviolet.
